# EUROPEAN PATENT APPLICATION

(11) **EP 3 957 337 A1**
(43) Date of publication of application: **23.02.2022**
(21) Application number: 20191822.4
(22) Date of filing: 19.08.2020
(51) Int. Cl.: A61L 9/20, A62B 18/04, A62B 18/08, A61B 5/00, A62B 9/00

(54) **PERSONAL AIR PURIFICATION APPARATUS**

(71) Applicant: HANIYA HOLDING LIMITED, 3032 Limassol (CY)
(72) Inventor: YAMANI, Hani Ahmad Z, Bahar ic-Caghaq, NXR_5130 (MT)
(74) Representative: Cornuejols, Georges

(57) **Abstract**

The personal air purification apparatus (100), characterized in that it comprises:
- a wearable air purification module (200) comprising:
- an entry (205) for an air flow,
- a continuous positive airway pressuring means (210),
- an ultraviolet light emitter (215) associated to a titanium oxide photocatalyst configured to purify the airflow and
- an output (220) for purified air
- a sealed face mask (300) comprising an intake (305) for purified air associated to the output of the wearable module. The apparatus may comprise an arm band (400) comprising a computing unit, a display and a communicating means configured to interact with the face mask and/or the wearable air purification module.

## Description

### TECHNICAL FIELD OF THE INVENTION

The present invention relates to a personal air purification apparatus. It applies, in particular, to the field of prevention of airborne diseases and viruses.

### BACKGROUND OF THE INVENTION

Airborne diseases and viruses pose a serious threat to the health and organization of human societies. To combat such a threat, preventive and palliative solutions are researched.

One such preventive measure consists for citizens to wear a face mask, disposable or not. However, the efficiency of such masks is insufficient, in particular when considering persons at greater health risk from the diseases or viruses.

### SUMMARY OF THE INVENTION

The present invention is intended to remedy all or part of these disadvantages.

To this effect, according to a first aspect, the present invention aims at a personal air purification apparatus, comprising:
- a wearable air purification module comprising:
   - an entry for an air flow,
   - a continuous positive airway pressuring means,
   - an ultraviolet light emitter associated to a titanium oxide photocatalyst configured to purify the airflow and
   - an output for purified air
- a sealed face mask comprising an intake for purified air associated to the output of the wearable module.

Such provisions allow for the reliable purification of air destined for the wearer of the apparatus.

In particular embodiments, the wearable module comprises an energy storing device configured to power said apparatus.

Such embodiments allow for the mobile use of the apparatus without relying on the capacity to plug into an energy network.

In particular embodiments, the wearable module comprises a control module configured at least to connect to a wireless data network.

Such embodiments allow for the remote monitoring of the status of the apparatus and the supply of data to the apparatus.

In particular embodiments, the wearable module comprises an outlet for power supply, said power supply being configured to supply the face mask.

Such embodiments allow for the remote monitoring of the status of the face mask and the supply of data to the face mask while carrying the power supply upon the back of the wearer, for example.

In particular embodiments, the face mask comprises an infrared thermometer configured to face inward of the face mask.

Such embodiments allow for the monitoring of the health status of the wearer to detect symptoms such as fever that might be troubling the wearer.

In particular embodiments, the face mask comprises an infrared thermometer configured to face outward of the face mask.

Such embodiments allow for the monitoring of the health status of individuals in the proximity of the wearer to evaluate health risks associated with being in the vicinity of said individuals.

In particular embodiments, the face mask comprises a camera facing outward of the face mask.

In particular embodiments, the activation of the camera is triggered as a function of the temperature captured by the infrared thermometer.

Such embodiments allow for the easing of contact tracing of infectious individuals by providing the picture of said individuals to the authorities.

In particular embodiments, the apparatus object of the present invention comprises a purified airflow quality and/or pressure measurement means.

Such embodiments allow for the monitoring of the status of the purifying means in order, for example, to alert the wearer in the event of malfunction.

In particular embodiments, the face mask comprises a head-up display unit.

Such embodiments allow for alerting the wearer of a particular status requiring their attention.

In particular embodiments, the head-up display unit is configured to display information relative to:
- a purified airflow quality and/or pressure value,
- an energy storing device level and/or
- a temperature measurement value.

In particular embodiments, the apparatus object of the present invention comprises an arm band comprising a computing unit, a display and a communicating means configured to interact with the face mask and/or the wearable air purification module.

Such embodiments allow for the display of data in a convenient manner to the wearer of the apparatus.

In particular embodiments, the arm band is configured to display a message upon the display as a function of the temperature captured.

In particular embodiments, the arm band is configured to display a message upon the display as a function of the temperature captured.

In particular embodiments, the arm band is configured to display a message upon the display as a function of the airflow quality and/or pressure measured.

### BRIEF DESCRIPTION OF THE DRAWINGS

Other advantages, purposes and particular characteristics of the invention shall be apparent from the following non-exhaustive description of at least one particular apparatus which is the object of this invention, in relation to the drawings annexed hereto, in which:
The figure 1 represents, schematically, a side view of a particular embodiment of the apparatus object of the present invention,

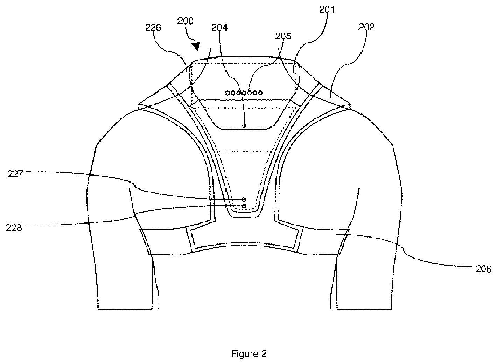

The figure 2 represents, schematically, a rear view of a particular embodiment of the wearable module of a particular embodiment of the apparatus object of the present invention,

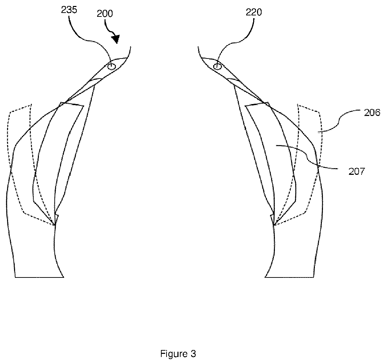

The figure 3 represents, schematically, a front view of a particular embodiment of the wearable module of a particular embodiment of the apparatus object of the present invention,

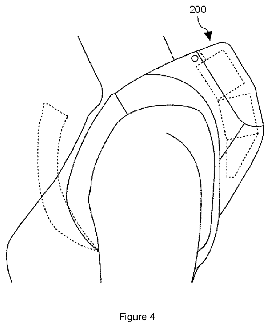

The figure 4 represents, schematically, a side view of a particular embodiment of the wearable module of a particular embodiment of the apparatus object of the present invention,

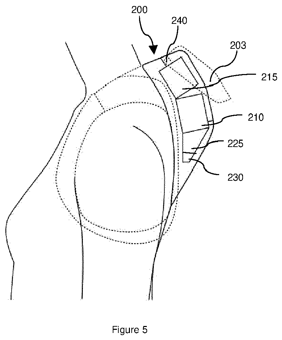

The figure 5 represents, schematically, a side view of the inside of a particular embodiment of the wearable module of a particular embodiment of the apparatus object of the present invention,

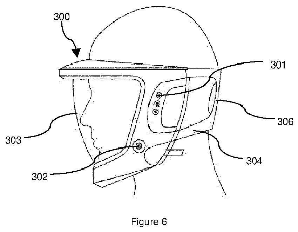

The figure 6 represents, schematically, a side view of a particular embodiment of the face mask of a particular embodiment of the apparatus object of the present invention,

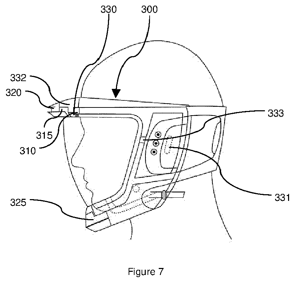

The figure 7 represents, schematically, a side view of the inside of a particular embodiment of the face mask of a particular embodiment of the apparatus object of the present invention,

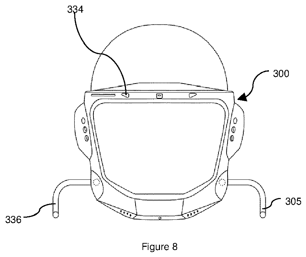

The figure 8 represents, schematically, a front view of a particular embodiment of the face mask of a particular embodiment of the apparatus object of the present invention,

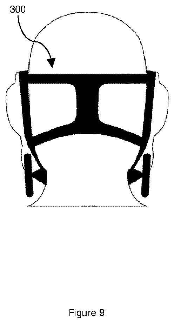

The figure 9 represents, schematically, a back view of a particular embodiment of the face mask of a particular embodiment of the apparatus object of the present invention,

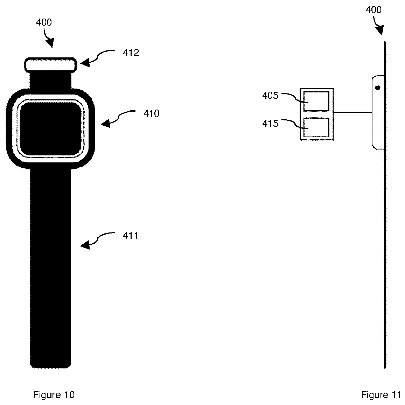

The figure 10 represents, schematically, a front view of a particular embodiment of the arm band of a particular embodiment of the apparatus object of the present invention and
The figure 11 represents, schematically, a side view of a particular embodiment of the arm band of a particular embodiment of the apparatus object of the present invention.

### DETAILED DESCRIPTION OF THE INVENTION

This description is not exhaustive, as each feature of one embodiment may be combined with any other feature of any other embodiment in an advantageous manner.

It should be noted at this point that the figures are not to scale.

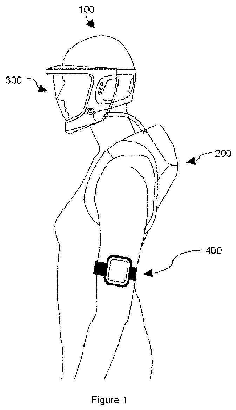

Figure 1 represents, schematically, a particular embodiment of the apparatus 100 object of the present invention. This personal air purification apparatus 100 comprises:
- a wearable air purification module 200 comprising:
   - an entry 205 for an air flow,
   - a continuous positive airway pressuring means 210,
   - an ultraviolet light emitter 215 associated to a titanium oxide photocatalyst configured to purify the airflow and
   - an output 220 for purified air
- a sealed face mask 300 comprising an intake 305 for purified air associated to the output of the wearable module.

The wearable air purification module 200 is configured to be worn upon a determined body part of the wearer, such as the back, a thigh, or an arm. The module preferably comprises a casing 201 configured to protect the functional elements of the module 200. Such casing 201 is formed in carbon fiber and positioned inside formed foam 202, for example.

Such casing 201 may comprise a hatch 203 preferably comprising a hatch lock 204. Such a hatch 203 allows access to the functional elements for maintenance purposes, for instance.

If the wearable air purification module 200 is intended to be worn upon a wearer's back, the module 200 comprises fixtures to the wearer, such as a strap 206 configured to surround the chest of the wearer. This strap 206 can be made to surround the shoulders of the wearer. In particular embodiments, the strap 206 comprises an magnetic locking mechanism 207 in which, for example, the strap 206 is divided into two parts, each part being magnetically attracted by the other in such a way that, once in contact, the two parts form the strap 206.

The strap 206 can be padded in particular locations to increase the comfort of the wearer.

The entry 205 for an air flow can be formed by vents in the casing 201 linked, via a conduit, to the continuous positive airway pressuring means 210.

In variants, the entry 205 is formed by inlets 301 located on the face mask 300, said inlets being linked via a conduit to the continuous positive airway pressuring means 210 of the module 200.

The continuous positive airway pressuring means 210 can be, for example, a continuous positive airway pressure pump. It should be noted here that continuous positive airway pressure (CPAP) is "a form of positive airway pressure (PAP) ventilation in which a constant level of pressure above atmospheric pressure is continuously applied to the upper airway" of the wearer.

The airflow is pushed, by the CPAP means to a purifying unit, comprising the ultraviolet light emitter 215 associated to a titanium oxide photocatalyst located within the unit.

The purified airflow is directed towards the output 220 which is, for example, an opening in the casing 201 destined to receive a conduit linking an inner volume of the face mask 300 to the output 220.

In particular embodiments, the module 200 comprises an energy storing device 225 configured to power said apparatus. Said energy storing device 225 can be, for instance, a rechargeable battery of any known type to a Person Skilled in the Art. Such an example is a polymer battery pack configured to last for up to 24 hours.

In variants, the module 200 further comprises a power switch 226 configured to activate the functional elements of the module 200 upon activation and deactivate the functional elements of the module 200 upon deactivation.

In variants, the module 200 further comprises a visual indicator 227 of a battery level of charge. Such a visual indicator 227 is a light emitting diode (LED), for example. In such an example, a parameter of light emission of the LED is a function of the battery level. Such a parameter can be, for example, intensity, color, and/or blinking frequency.

In variants, the module 200 further comprises a charging port 228 configured to link the energy storing device 225 to a power outlet.

In particular embodiments, the wearable module 200 comprises an outlet 235 for power supply, said power supply being configured to supply the face mask 300.

Such an outlet 235 is, for example, a socket upon the casing 201 of the module allowing for the plugging of a power cable of the face mask 300.

In particular embodiments, the wearable module 200 comprises a control module 230 configured at least to connect to a wireless data network.

The control module 230 is, for example, a microcontroller combining a processing unit and a computer memory. Such control module 230 is preferably configured to command the activation/deactivation of the continuous positive airway pressuring means 210 and of the ultraviolet light emitter 215.

The connection to the wireless data network can be performed with the use of a network card linked to the control module 230, for example. The wireless data network can be, for example, a wired network such as the Internet, accessible via Wi-Fi communication, or a mobile network such as a 4G or 5G.

The control module 230 is configured to emit and/or receive data to/from the data network, the nature of said data depending on the particular use case considered. For example, the control module 230 can send data relative to:
- a power level of the energy storing device 225 or
- functional parameters of either the continuous positive airway pressuring means 210 and of the ultraviolet light emitter 215.

In variants, the outlet 235 also serves as a data transmission port between the control module 230 and the face mask 300.

The face mask 300 is configured to be sealed, that is isolating a respiratory space for the wearer from the outside environment. Sealing the face mask 300 is performed by the attachment of the face mask 300 upon the head of the wearer using a fixture mechanism. Such a fixture mechanism is, for example, a belt 304 associated to a magnetic lock 306.

The face mask 300 comprises a visor 303, made from acrylic plastic with transparent epoxy resin for example.

The face mask 300 preferably comprises an exhaust 302 for the carbon dioxide emitted by the wearer of the apparatus 100.

In particular embodiments, the face mask 300 comprises an infrared thermometer 310 configured to face inward of the face mask.

In particular embodiments, the face mask 300 comprises an infrared thermometer 315 configured to face outward of the face mask.

In variants, a functional parameter of the ultraviolet light emitter 215 is determined as a function of the temperature measured by the thermometer 315. For example, if a person close to the wearer of the face mask 300 presents a temperature above a determined threshold representative of a risk of illness, the ultraviolet light emitter 215 may be set to a higher purifying quality or rate at the cost of battery life.

In particular embodiments, the face mask 300 comprises a camera 320 facing outward of the face mask. Such a camera 320 can be an IP camera.

In variants, a functional parameter of the ultraviolet light emitter 215 is determined as a function of at least one image captured by the camera 320. For example, the camera 320 can be configured to capture a stream of images, being analyzed by an analysis module, such as a computer software, to determine a number of people in the vicinity of the wearer. If the number of people near the wearer of the face mask 300 presents is above a determined threshold representative of a risk of transmission, the ultraviolet light emitter 215 may be set to a higher purifying quality or rate at the cost of battery life.

In variants, the activation of the camera 320 is dependent on a temperature captured by the external thermometer 315. In such variants, when the measured temperature of a person is above a predetermined value, the camera 320 is activated to capture an image of that person and the surrounding area. These variants allow for better contact tracing.

In variants, the apparatus 100 comprises a geolocation module, which can be a GPS module located within either the wearable module 200, the face mask 300, the arm band 400. In variants, the geolocation module is located within a mobile device belonging to the wearer of the apparatus 100, said device being connected to the apparatus via a control module 130. In such variants, when the measured temperature of a person is above a predetermined value, the geolocation of the apparatus is captured and stored and/or transmitted to a remote memory.

In particular embodiments, the face mask 300 comprises a speaker 325 configured to face outward of the face mask.

In particular embodiments, the apparatus 100 comprises a purified airflow quality and/or pressure measurement means 330.

Such a purified airflow quality measurement means 330 is, for example, a particle counter.

Such a pressure measurement means 330 is, for example, a pressure meter.

The purified airflow quality and/or pressure measurement means 330 can be located in the face mask 300 or the wearable module 200.

In particular embodiments, the face mask 300 comprises a light 334, such as a LED system to allow for visibility during nighttime or indoors, for example.

In particular embodiments, the face mask 300 comprises a data and/or power line 336 configured to link the face mask 300 to, respectively, an energy storing device 225 or the control module 230 of the wearable module 200.

In particular embodiments, the face mask 300 comprises a head-up display unit 335. This head-up display unit 335 can be formed of a projector configured to project upon the visor 303.

The head-up display 335 unit can be configured to display information relative to:
- a purified airflow quality and/or pressure value,
- an energy storing device level,
- a notification of arrival and/or the content of a new message or call on a handset associated with the apparatus 100 or received by a control module of the apparatus 100 and/or
- a temperature measurement value.

For example, the head-up display 335 can be configured to show an alert when nearby persons present temperatures higher than a predetermined threshold.

In variants, the face mask 300 comprises a wafer speaker 331. Such wafer speaker 331 can be used to receive audio signals from a telephone.

In variants, the face mask 300 comprises a control module 332. The functions and embodiments of said control module 332 are similar to the control module 230 of the wearable module 200.

In variants, the face mask 300 comprises a reading glass temple slot 333.

In particular embodiments, the apparatus 100 comprises an arm band 400 comprising a computing unit 405, a display 410 and a communicating means 415 configured to interact with the face mask 300 and/or the wearable air purification module 200.

The computing unit 405 is, for example, a microcontroller associated with a computer memory. In variants, the computing unit 405 is a microprocessor.

The display 410 is, for example, a liquid crystal display (LCD) screen.

The communicating means 415 is, for example, an antenna and the associated controller configured to emit and/or receive data to and/or from a data network such as the internet.

The arm band 400 can be positioned upon a wearers body by the use of a belt 411 and buckle 412 system, said belt 411 comprising, for example, a magnetic tape type of fixation mechanism.

In particular embodiments, the arm band 400 is configured to display a message upon the display 410 as a function of the temperature captured by the thermometer 315 facing outwards.

For instance, such a message could be an alert of the presence of a person presenting fever symptoms.

In particular embodiments, the arm band 400 is configured to display a message upon the display 410 as a function of the temperature captured by the thermometer 310 facing inwards.

For instance, such a message could be an alert that the wearer is presenting fever symptoms.

In particular embodiments, the arm band 400 is configured to display a message upon the display 410 as a function of the airflow quality and/or pressure measured.

For instance, such a message could be an alert that the oxygen pressure is low.

## Claims

1. Personal air purification apparatus (100), **characterized in that** it comprises:
- a wearable air purification module (200) comprising:
- an entry (205) for an air flow,
- a continuous positive airway pressuring means (210),
- an ultraviolet light emitter (215) associated to a titanium oxide photocatalyst configured to purify the airflow and
- an output (220) for purified air
- a sealed face mask (300) comprising an intake (305) for purified air associated to the output of the wearable module.

2. Apparatus (100) according to claim 1, in which the wearable module (200) comprises an energy storing device (225) configured to power said apparatus.

3. Apparatus (100) according to any one of claims 1 or 2, in which the wearable module (200) comprises a control module (230) configured at least to connect to a wireless data network.

4. Apparatus (100) according to any one of claims 1 to 3, in which the wearable module (200) comprises an outlet (235) for power supply, said power supply being configured to supply the face mask (300).

5. Apparatus (100) according to any one of claims 1 to 4, in which the face mask (300) comprises an infrared thermometer (310) configured to face inward of the face mask.

6. Apparatus (100) according to any one of claims 1 to 5, in which the face mask (300) comprises an infrared thermometer (315) configured to face outward of the face mask.

7. Apparatus (100) according to any one of claims 1 to 6, in which the face mask (300) comprises a camera (320) facing outward of the face mask.

8. Apparatus (100) according to claim 5 and 7, in which the activation of the camera (320) is triggered as a function of the temperature captured by the infrared thermometer (315).

9. Apparatus (100) according to any one of claims 1 to 8, comprising a purified airflow quality and/or pressure measurement means (240).

10. Apparatus (100) according to any one of claims 1 to 8, in which the face mask (300) comprises a head-up display unit (330).

11. Apparatus (100) according to claim 10, in which the head-up display (330) unit is configured to display information relative to:
- a purified airflow quality and/or pressure value,
- an energy storing device level and/or
- a temperature measurement value.

12. Apparatus (100) according to any one of claims 1 to 11, comprising an arm band (400) comprising a computing unit (405), a display (410) and a communicating means (415) configured to interact with the face mask (300) and/or the wearable air purification module (200).

13. Apparatus (100) according to claims 5 and 12, in which the arm band (400) is configured to display a message upon the display (410) as a function of the temperature captured.

14. Apparatus (100) according to claims 6 and 12, in which the arm band (400) is configured to display a message upon the display (410) as a function of the temperature captured.

15. Apparatus (100) according to claims 9 and 12, in which the arm band (400) is configured to display a message upon the display (410) as a function of the airflow quality and/or pressure measured.
